# EUROPEAN PATENT SPECIFICATION

(11) **EP 2 268 266 B1**
(45) Date of publication and mention of the grant of the patent: **21.10.2020**
(21) Application number: 09721857.2
(22) Date of filing: 19.03.2009
(51) Int. Cl.: A61K 31/365, A61K 9/14, A61K 9/48

(54) **PROCESS FOR OBTAINING POWDER COMPOSITIONS OF ORLISTAT TO BE FILLED INTO A CAPSULE**
VERFAHREN ZUR GEWINNUNG VON PULVERZUSAMMENSETZUNGEN VON ORLISTAT ZUM FÜLLEN EINER KAPSEL
PROCÉDÉ POUR OBTENIR DES COMPOSITIONS EN POUDRE D'ORLISTAT À REMPLIR DANS UNE CAPSULE

(30) Priority: 20.03.2008 PL 38475608
(43) Date of publication of application: 05.01.2011
(60) Divisional application: 20160560.7
(73) Proprietor: Zaklady Farmaceutyczne "Polpharma" S.A., 83-200 Starogard Gdanski (PL)
(72) Inventor: MAZGALSKI, Jaroslaw, PL-83-010 Straszyn (PL); SZEMECKA-GOLABEK, Anna, PL-83-212 Dabrowka (PL); KAMINSKA, Anna, PL-83-200 Starogard Gdanski (PL)
(74) Representative: Isarpatent
(86) International application number: PCT/PL2009/000025
(87) International publication number: WO 2009/116880

(56) References cited:
- WO-A-2009/039157
- WO-A-2009/044380
- CL-A- 2007 702 287
- DE-A1- 10 021 618
- US-A1- 2004 126 424
- US-A1- 2006 269 510

## Description

The invention relates to a novel process for the manufacture of stable, meeting relevant quality requirements pharmaceutical compositions in the form of encapsulated powder comprising Orlistat and pharmaceutically acceptable additives. The disclosure further relates to pharmaceutical compositions of Orlistat in the form of encapsulated powder obtained in accordance with the process of the present invention.

Orlistat, known as tetrahydrolipstatin or THL, is an inhibitor of pancreatic lipase. Orlistat acts within the GI tract to inhibit the enzymes which hydrolyze dietary fat to glycerol and fatty acids and hence prevents absorption of lipids into the bloodstream. For this reason, Orlistat is indicated for the treatment of obesity in patients with body mass index (BMI) of greater or equal to 30 kg/m².

Due to distinct physicochemical properties, namely waxy character, melting point at 44 °C and low chemical stability, Orlistat is considered in the state of art as a technologically demanding and difficult to handle active ingredient. Known in the state of art methods for obtaining pharmaceutical compositions comprising Orlistat employ complicated and expensive technologies, for instance, extrusion and spheronisation disclosed in International patent application WO 98/34607 or expansion of a solution or homogeneous dispersion comprising Orlistat under reduced pressure without boiling disclosed in International patent application WO 02/00201. Similarly, known in the state of art pharmaceutical compositions comprising Orlistat teach to employ in the formulations of the active ingredient rarely used in practice, atypical, expensive and often unstable pharmaceutical additives in order to obtain a product meeting relevant quality requirements. Such atypical pharmaceutical additives are, for example, fatty acid esters of polyols disclosed in International patent application WO 01/19378, sucrose fatty acid esters disclosed in International patent application WO 02/098412, fatty acids or fatty acid salts or mixtures of fatty acids and fatty acid salts disclosed in International patent application WO 02/098413, bile acid sequestrants disclosed in International patent application WO 02/09815, poorly digestible, poorly fermentable, hydrophilic and/or hydrocolloidal food grade thickeners disclosed in International patent application WO 00/09122 and complex surfactants disclosed in International patent application WO 01/19340.

Chilean patent application CL200702287 discloses a process for manufacturing pharmaceutical compositions of Orlistat comprising the following steps:
a) mixing Orlistat with a first amount of excipients;b) milling the mixture obtained in step a);
c) mixing a second amount of excipients with the mixture obtained in step b); and
d) encapsulating the mixture obtained in step c) in hard gelatin capsules.

The above mentioned publications clearly indicate the need of employing in the manufacture of pharmaceutical compositions of Orlistat complicated technologies and/or , atypical, often rendering technological difficulties and expensive pharmaceutical additives. Also Roche's reference product, Xenical®, manufactured using extrusion and spheronisation method, which is regarded as a relatively complicated, time-consuming, demanding supervision of experienced technologists and expensive technology, is a strong confirmation of the discussed presumption.

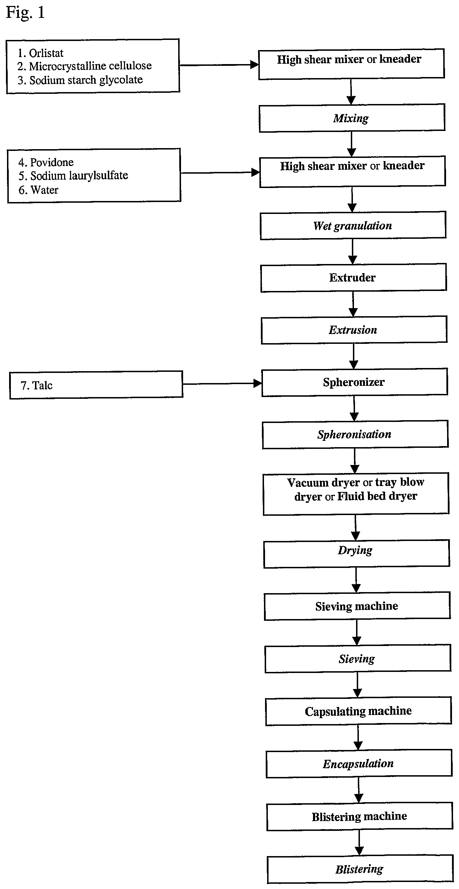

The extrusion and spheronisation technology employed by Roche in the manufacture of Xenical® comprises numerous critical steps which have direct influence on chemical purity and final quality of the dosage form. These critical steps are, for example, wet granulation stage (due to the fact that Orlistat readily undergoes hydrolysis) and extrusion stage, which is accompanied by temperature rise (since Orlistat readily oxidizes and has melting point at temperature as low as 44 °C). A scheme of the manufacturing process of Roche's Xenical® by extrusion and spheronisation method is presented in Fig. 1.

In addition, the formulation of Roche's Xenical® comprises micronized Orlistat, i.e. having greater total surface when compared to unmicronized active ingredient. As a result of this feature, the active ingredient present in the formulation of Xenical® is much more prone to the above indicated undesired phenomena. Therefore, to reduce their influence on the manufacturing process of Xenical®, the process requires performing in non-standard conditions, for instance some stages of the process are carried out under inert atmosphere or under vacuum. For this reason, the discussed manufacturing process is regarded as strongly complicated and expensive.

Surprisingly, it was found that pharmaceutical capsule compositions of Orlistat can be manufactured using relatively simple, inexpensive and fast process according to claim 1 comprising blending Orlistat and pharmaceutically acceptable additives and encapsulating thus obtained pharmaceutical powder composition without the need of employing in the composition atypical, often rendering technological difficulties and expensive pharmaceutical additives. It was also found that the process according to the present invention provides pharmaceutical powder compositions comprising Orlistat of higher chemical stability than Xenical®. The above feature is an effect of better protection of the active ingredient Orlistat against chemical degradation in the composition obtained by the process according to the invention.

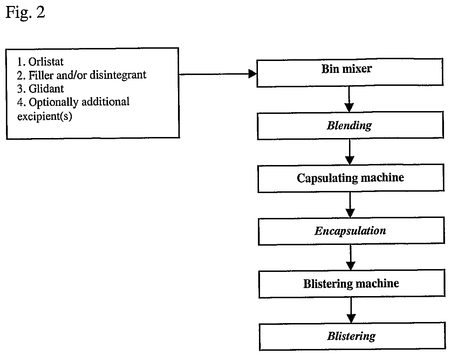

The invention relates to a process for the manufacture of pharmaceutical compositions in the form of encapsulated powder comprising Orlistat and pharmaceutically acceptable additives which comprises two steps, a first step of obtaining pharmaceutical powder by blending Orlistat and pharmaceutically acceptable additives and a next, second step of encapsulating thus obtained powder. In the first step of the process according to the invention Orlistat is blended with pharmaceutically acceptable additives selected from the group comprising a glidant and a disintegrant or a filler or, preferably, a mixture of a disintegrant and a filler and, optionally, one or more additional pharmaceutically acceptable additives to obtain a homogeneous powder. In the second step of the process the homogeneous powder obtained in the first step is encapsulated. A scheme of the manufacturing process of pharmaceutical compositions comprising Orlistat according to the invention is presented in Fig. 2.

The process for the manufacture of pharmaceutical compositions comprising Orlistat according to the invention does not require employing atypical, often rendering technological difficulties and expensive pharmaceutical additives, the use of which is implied in the prior art. The process according to the invention allows for employing in the manufacture of pharmaceutical composition comprising Orlistat typical, widely used in practice pharmaceutically acceptable additives , which greatly simplifies the manufacturing process and reduces costs.

In a preferred embodiment of the invention, the pharmaceutical powder composition manufactured according to the process of the invention comprises Orlistat in an amount of 30 to 70% by weight of the powder composition. In another preferred embodiment of the invention, the pharmaceutical powder composition manufactured according to the process of the invention comprises Orlistat in an amount of 120 mg per dose. In yet another preferred embodiment of the invention, the pharmaceutical powder composition manufactured according to the process of the invention comprises Orlistat in an amount of 60 mg per dose.

The process for the manufacture of pharmaceutical powder compositions comprising Orlistat according to the invention does not encompass employing in the process micronized active ingredient. The above is clearly an advantage of the process according to the invention due to significant technological difficulties with micronizing Orlistat resulting from its waxy character. In addition, the process of the invention does not require employing in the powder compositions Orlistat active ingredient of a specific and narrow range of particle size, in particular, the process does not require eliminating from the compositions largest particles present in commercially available Orlistat, i.e. particles of size in the range of 200 to 300 µm. In accordance to the above, the process of the invention involves employing in the powder composition Orlistat active ingredient having a D₉₀ particle size of more than 30 µm.

In a preferred embodiment of the invention, the pharmaceutical powder composition manufactured according to the process of the invention comprises a glidant in an amount of 1 to 10% by weight of the powder composition. In another preferred embodiment of the invention, the pharmaceutical powder composition comprises a glidant selected from the group consisting of anhydrous silica dioxide, talc, magnesium stearate , calcium stearate, stearic acid, sodium stearyl fumarate, glyceryl behenate, glyceryl palmitostearate, sodium laurylsulfate and mixtures thereof.

The process for the manufacture of pharmaceutical powder compositions comprising Orlistat according to the invention involves employing in the composition a disintegrant or a filler or a mixture of a disintegrant and a filler. In a preferred embodiment of the invention, the powder composition comprises a mixture of a disintegrant and a filler.

In a preferred embodiment of the invention, the pharmaceutical powder composition manufactured according to the process of the invention comprises a disintegrant in an amount of 3 to 30% by weight of the powder composition. In another preferred embodiment of the invention, the pharmaceutical powder composition comprises a disintegrant selected from the group consisting of sodium starch glycolate, croscarmellose, crospovidone and mixtures thereof.

In a preferred embodiment of the invention, the pharmaceutical powder composition manufactured according to the process of the invention comprises a filler in an amount of 10 to 80% by weight of the powder composition. In another preferred embodiment of the invention, the pharmaceutical powder composition comprises a filler selected from the group consisting of microcrystalline cellulose, silicified microcrystalline cellulose, powdered cellulose, maize starch, modified maize starch, potato starch, lactose monohydrate, lactose anhydrous, mannitol, dibasic calcium phosphate dihydrate, anhydrous dibasic calcium phosphate, magnesium carbonate, erythritol, trehalose and mixtures thereof. In yet another preferred embodiment of the invention, the pharmaceutical powder composition comprises a filler selected from the group consisting of low moisture content microcrystalline cellulose, low moisture content maize starch, low moisture content potato starch and mixtures thereof.

Low moisture content fillers mentioned above are characterized by reduced free water content when compared to standard fillers of the same type. For instance, low moisture content microcrystalline cellulose having free water content of less than 1,5% is available from JRS Pharma under the trade name Vivapur PH 112, low moisture content corn starch having free water content of 7,5% is available from Roquette under the trade name Corn starch 400L NF, low moisture content potato starch having free water content of 8,0% is available from Roquette under the trade name Potato starch 8,0%.

In a preferred embodiment of the invention, the pharmaceutical powder composition manufactured according to the process of the invention comprises one or more additional pharmaceutically acceptable additives. In another preferred embodiment of the invention, the pharmaceutical powder composition comprises a surfactant as the additional pharmaceutically acceptable additive present in the powder composition in an amount of 0,1 to 5% by weight. In yet another preferred embodiment of the invention, the surfactant employed as the additional pharmaceutically acceptable additive is sodium lauryl sulfate.

The following examples illustrate various aspects of the present invention. They are not to be construed to limit the claims in any manner whatsoever.

### Example 1

Manufacturing process of encapsulated powder composition comprising Orlistat for a batch size of 3,45 kg (15 000 capsules, 230 mg each) comprising the following ingredients.

| **Ingredient** | **Amount [kg]** | **Amount [mg/caps.]** | **% per caps.** |
|---|---|---|---|
| Orlistat | 1,800 | 120,00 | 52,17 |
| Vivapur PH 112 | 0,856 | 57,10 | 24,83 |
| Sodium starch glycolate type A | 0,621 | 41,40 | 18,00 |
| Anhydrous silica dioxide | 0,138 | 9,20 | 4,00 |
| Sodium lauryl sulfate | 0,035 | 2,30 | 1,00 |
| **TOTAL** | **3,450** | **230,00** | **100,00** |

The above ingredients in given amounts were passed through a sieve mesh size 1,0 mm and blended in a 20-litre bin mixer for 30 minutes at the speed of 12 rpm until a homogeneous powder mixture was obtained. The powder composition was next encapsulated into capsules size 1 using rotary capsulating machine. Hard gelatin capsule composition comprising 120 mg of Orlistat in 230 mg of powder was obtained.

### Example 2

Manufacturing process of encapsulated powder composition comprising Orlistat for a batch size of 3,45 kg (30 000 capsules, 115 mg each) comprising the following ingredients.

| **Ingredient** | **Amount [kg]** | **Amount [mg/caps.]** | **% per capsule** |
|---|---|---|---|
| Orlistat | 1,800 | 60,00 | 52,17 |
| Mannitol | 0,930 | 31,00 | 26,96 |
| Crospovidone | 0,345 | 11,50 | 10,00 |
| Corn starch 400L NF | 0,2025 | 6,75 | 5,87 |
| Anhydrous silica dioxide | 0,138 | 4,60 | 4,00 |
| Sodium lauryl sulfate | 0,0345 | 1,15 | 1,00 |
| **TOTAL** | **3,450** | **115,00** | **100,00** |

The above ingredients in given amounts were passed through a sieve mesh size 1,0 mm and blended in a 20-litre bin mixer for 30 minutes at the speed of 12 rpm until a homogeneous powder mixture was obtained. The powder composition was next encapsulated into capsules size 3 using rotary capsulating machine. Hard gelatin capsule composition comprising 60 mg of Orlistat in 115 mg of powder was obtained.

### Example 3

Manufacturing process of encapsulated powder composition comprising Orlistat for a batch size of 3,45 kg (15 000 capsules, 230 mg each) comprising the following ingredients.

| **Ingredient** | **Amount [kg]** | **Amount [mg/caps.]** | **% per capsule** |
|---|---|---|---|
| Orlistat | 1,800 | 120,00 | 52,17 |
| Dibasic calcium phosphate dihydrate | 0,7385 | 49,23 | 21,41 |
| Sodium starch glycolate type A | 0,739 | 49,27 | 21,42 |
| Anhydrous silica dioxide | 0,138 | 9,20 | 4,00 |
| Sodium lauryl sulfate | 0,0345 | 2,30 | 1,00 |
| **TOTAL** | **3,450** | **230,00** | **100,00** |

The above ingredients in given amounts were passed through a sieve mesh size 1,0 mm and blended in a 20-litre bin mixer for 30 minutes at the speed of 12 rpm until a homogeneous powder mixture was obtained. The powder composition was next encapsulated into capsules size 1 using rotary capsulating machine. Hard gelatin capsule composition comprising 120 mg of Orlistat in 230 mg of powder was obtained.

### Example 4

Manufacturing process of encapsulated powder composition comprising Orlistat for a batch size of 3,2 kg (20 000 capsules, 160 mg each) comprising the following ingredients.

| **Ingredient** | **Amount [kg]** | **Amount [mg/caps.]** | **% per capsule** |
|---|---|---|---|
| Orlistat | 2,400 | 120,00 | 75,00 |
| Lactose anhydrous | 0,512 | 25,60 | 16,00 |
| Sodium starch glycolate type A | 0,160 | 8,00 | 5,00 |
| Anhydrous silica dioxide | 0,064 | 3,20 | 2,00 |
| Sodium lauryl sulfate | 0,064 | 3,20 | 2,00 |
| **TOTAL** | **3,200** | **160,00** | **100,00** |

The above ingredients in given amounts were passed through a sieve mesh size 1,0 mm and blended in a 20-litre bin mixer for 30 minutes at the speed of 12 rpm until a homogeneous powder mixture was obtained. The powder composition was next encapsulated into capsules size 2 using rotary capsulating machine. Hard gelatin capsule composition comprising 120 mg of Orlistat in 160 mg of powder was obtained.

### Example 5

Manufacturing process of encapsulated powder composition comprising Orlistat for a batch size of 3,45 kg (15 000 capsules, 230 mg each) comprising the following ingredients.

| **Ingredient** | **Amount [kg]** | **Amount [mg/caps.]** | **% per capsule** |
|---|---|---|---|
| Orlistat | 1,800 | 120,00 | 52,17 |
| Corn starch 400L NF | 0,856 | 57,10 | 24,83 |
| Sodium starch glycolate type A | 0,621 | 41,40 | 18,00 |
| Anhydrous silica dioxide | 0,138 | 9,20 | 4,00 |
| Sodium lauryl sulfate | 0,035 | 2,30 | 1,00 |
| **TOTAL** | **3,450** | **230,00** | **100,00** |

The above ingredients in given amounts were passed through a sieve mesh size 1,0 mm and blended in a 20-litre bin mixer for 30 minutes at the speed of 12 rpm until a homogeneous powder mixture was obtained. The powder composition was next encapsulated into capsules size 1 using rotary capsulating machine. Hard gelatin capsule composition comprising 120 mg of Orlistat in 230 mg of powder was obtained.

### Example 6

Manufacturing process of encapsulated powder composition comprising Orlistat for a batch size of 3,45 kg (30 000 capsules, 115 mg each) comprising the following ingredients.

| **Ingredient** | **Amount [kg]** | **Amount [mg/caps.]** | **% per capsule** |
|---|---|---|---|
| Orlistat | 1,800 | 60,00 | 52,17 |
| Corn starch 400L NF | 0,856 | 28,55 | 24,83 |
| Sodium starch glycolate type A | 0,621 | 20,70 | 18,00 |
| Anhydrous silica dioxide | 0,138 | 4,60 | 4,00 |
| Sodium lauryl sulfate | 0,035 | 1,15 | 1,00 |
| **TOTAL** | **3,450** | **115,00** | **100,00** |

The above ingredients in given amounts were passed through a sieve mesh size 1,0 mm and blended in a 20-litre bin mixer for 30 minutes at the speed of 12 rpm until a homogeneous powder mixture was obtained. The powder composition was next encapsulated into capsules size 3 using rotary capsulating machine. Hard gelatin capsule composition comprising 60 mg of Orlistat in 115 mg of powder was obtained.

The invention relates also to pharmaceutical compositions in the form of encapsulated powder comprising Orlistat obtained in accordance with the process of the present invention.

Pharmaceutical compositions in the form of encapsulated powder comprising Orlistat obtained in accordance with the process of the present invention effectively protect Orlistat, which is regarded as a highly chemically unstable active ingredient, against chemical degradation. The above is well demonstrated by comparing results of the stability tests of the composition prepared in accordance with the process of the present invention presented in example 1 and the reference product, Roche's Xenical®.

Testing of chemical stability of pharmaceutical compositions comprising Orlistat is carried out by measuring the increase of content of the main product of degradation of Orlistat, i.e. 5-[(N-formylleucyl)-oxy]-2-hexyl-3-hydroxyhexadecanic acid, also known as delactone, in time. The results of stability tests performed after storing both tested products in the conditions of, respectively, the temperature at 40 °C and 75% relative humidity and the temperature at 30 °C and 65% relative humidity are given below.

| **Delactone content** | **Storing conditions: 40 °C and 75% rh** | | | |
|---|---|---|---|---|
| | T₀ | 1 month | 2 months | 3 months |
| **Composition of example** 1 **in PVDC packaging** | BRT | 0,33 | 0,97 | 3,26 |
| **Xenical®** | 0,39 | 0,89 | 3,88 | 7,22 |

| | | | | |
|---|---|---|---|---|
| BRT - below reporting threshold | | | | |

| **Delactone content** | **Storing conditions: 30 °C and 65% rh** | | | | |
|---|---|---|---|---|---|
| | T₀ | 1 month | 2 months | 3 months | 6 months |
| **Composition of example 1 in PVDC packaging** | BRT | BRT | BRT | BRT | 0,11 |
| **Xenical®** | 0,39 | 0,44 | 0,49 | 0,54 | 0,65 |

| | | | | | |
|---|---|---|---|---|---|
| BRT - below reporting threshold | | | | | |

The above presented stability data clearly demonstrate that pharmaceutical compositions in the form of encapsulated powder comprising Orlistat and typical, widely used pharmaceutically acceptable additives manufactured in accordance with the relatively simple and inexpensive process of the present invention have significantly higher chemical stability than the reference product Xenical®, which is manufactured using complicated and expensive extrusion and spheronisation method.

The invention relates also to the use of Orlistat having a D₉₀ particle size of more than 30 µm in the manufacture of pharmaceutical powder compositions by means of blending Orlistat and pharmaceutically acceptable additives selected from the group comprising a glidant and a disintegrant or a filler or, preferably, a mixture of a disintegrant and a filler as defined above.

## Claims

1. A process for the manufacture of pharmaceutical compositions in the form of encapsulated powder comprising Orlistat and pharmaceutically acceptable additives selected from the group comprising a glidant and one or both of a filler and a disintegrant, **characterized in that** the process comprises two steps: a first step of obtaining pharmaceutical powder by blending Orlistat and pharmaceutically acceptable additives and a next, second step of encapsulating thus obtained powder.

2. The process according to claim 1, wherein Orlistat is present in the powder composition in an amount of 30 to 70% by weight.

3. The process according to claim 1, wherein the glidant is present in the powder composition in an amount of 1 to 10% by weight.

4. The process according to claim 1, wherein the disintegrant is present in the powder composition in an amount of 3 to 30% by weight.

5. The process according to claim 4, wherein the disintegrant is selected from the group consisting of sodium starch glycolate, croscarmellose, crospovidone and mixtures thereof.

6. The process according to claim 1, wherein the filler is present in the powder composition in an amount of 10 to 80% by weight.

7. The process according to claim 1, wherein the powder composition comprises one or more additional pharmaceutically acceptable additives.

8. The process according to claim 7, wherein the additional pharmaceutically acceptable additive is a surfactant present in the powder composition in an amount of 0,1 to 5% by weight.

9. A pharmaceutical composition in the form of encapsulated powder comprising Orlistat manufactured according to a process as defined in any of the claims 1 to 8.

10. The use of Orlistat in a process for the manufacture of pharmaceutical compositions in the form of encapsulated powder wherein the process of manufacture comprises a first step of blending Orlistat and pharmaceutically acceptable additives selected from the group comprising a glidant and one or both of a filler and a disintegrant, and a next, second step of encapsulating the blend.

11. The use of Orlistat according to claim 10, wherein Orlistat is present in the powder composition in an amount of 30 to 70% by weight.

12. The use of Orlistat according to claim 10, wherein the glidant is present in the powder composition in an amount of 1 to 10% by weight and is selected from the group consisting of anhydrous silica dioxide, talc, magnesium stearate , calcium stearate, stearic acid, sodium stearyl fumarate, glyceryl behenate, glyceryl palmitostearate, sodium laurylsulfate and mixtures thereof.

13. The use of Orlistat according to claim 10, wherein the disintegrant is present in the powder composition in an amount of 3 to 30% by weight and is selected from the group consisting of sodium starch glycolate, croscarmellose, crospovidone and mixtures thereof.

14. The use of Orlistat according to claim 10, wherein the filler is present in the powder composition in an amount of 10 to 80% by weight and is selected from the group consisting of microcrystalline cellulose, silicified microcrystalline cellulose, powdered cellulose, maize starch, modified maize starch, potato starch, lactose monohydrate, lactose anhydrous, mannitol, dibasic calcium phosphate dihydrate, anhydrous dibasic calcium phosphate, magnesium carbonate, erythritol, trehalose and mixtures thereof, preferably low moisture content microcrystalline cellulose, low moisture content maize starch, low moisture content potato starch or a mixture thereof.

15. The use of Orlistat according to claim 10, wherein the powder composition comprises one or more additional pharmaceutically acceptable additives, preferably a surfactant present in the composition in an amount of 0,1 to 5% by weight, preferably sodium lauryl sulfate.

## Patentansprüche

1. Verfahren zur Herstellung von pharmazeutischen Zusammensetzungen in Form eines eingekapselten Pulvers, umfassend Orlistat und pharmazeutisch annehmbare Additive, ausgewählt aus der Gruppe, bestehend aus einem Gleitmittel und einem oder beiden aus einem Füllstoff und einem Sprengmittel, **gekennzeichnet dadurch, dass** das Verfahren zwei Schritte umfasst: einen ersten Schritt des Gewinnens eins pharmazeutischen Pulvers durch Mischen von Orlistat und pharmazeutisch annehmbaren Additiven und einen nächsten, zweiten Schritt des Einkapselns des auf diese Weise erhaltenen Pulvers.

2. Verfahren nach Anspruch 1, wobei Orlistat in der Pulverzusammensetzung in einer Menge von 30 bis 70 Gew.-% vorliegt.

3. Verfahren nach Anspruch 1, wobei das Gleitmittel in der Pulverzusammensetzung in einer Menge von 1 bis 10 Gew.-% vorliegt.

4. Verfahren nach Anspruch 1, wobei das Sprengmittel in der Pulverzusammensetzung in einer Menge von 3 bis 30 Gew.-% vorliegt.

5. Verfahren nach Anspruch 4, wobei das Sprengmittel ausgewählt ist aus der Gruppe, bestehend aus Natriumstärkeglykolat, Croscarmellose, Crospovidon und Mischungen davon.

6. Verfahren nach Anspruch 1, wobei der Füllstoff in der Pulverzusammensetzung in einer Menge von 10 bis 80 Gew.-% vorliegt.

7. Verfahren nach Anspruch 1, wobei die Pulverzusammensetzung ein oder mehrere zusätzliche pharmazeutisch annehmbare Additive umfasst.

8. Verfahren nach Anspruch 7, wobei das zusätzliche pharmazeutisch annehmbare Additiv ein Tensid ist, das in der Pulverzusammensetzung in einer Menge von 0,1 bis 5 Gew.-% vorhanden ist.

9. Pharmazeutische Zusammensetzung in Form eines eingekapselten Pulvers, umfassend Orlistat, hergestellt nach einem Verfahren nach einem der Ansprüche 1 bis 8.

10. Verwendung von Orlistat in einem Verfahren zur Herstellung von pharmazeutischen Zusammensetzungen in der Form eines eingekapselten Pulvers, wobei das Herstellungsverfahren einen ersten Schritt des Mischens von Orlistat und pharmazeutisch annehmbaren Additiven, ausgewählt aus der Gruppe, die ein Gleitmittel und eines oder beide aus einem Füllstoff und einem Sprengmittel umfassen, und einen nächsten, zweiten Schritt des Einkapselns der Mischung umfasst.

11. Verwendung von Orlistat nach Anspruch 10, wobei Orlistat in der Pulverzusammensetzung in einer Menge von 30 bis 70 Gew.-% vorliegt.

12. Verwendung von Orlistat nach Anspruch 10, wobei das Gleitmittel in der Pulverzusammensetzung in einer Menge von 1 bis 10 Gew.-% vorliegt und ausgewählt ist aus der Gruppe, bestehend aus wasserfreiem Siliciumdioxid, Talk, Magnesiumstearat, Calciumstearat, Stearinsäure, Natriumstearylfumarat, Glycerylbehenat, Glycerylpalmitostearat, Natriumlaurylsulfat und Mischungen davon.

13. Verwendung von Orlistat nach Anspruch 10, wobei das Sprengmittel in der Pulverzusammensetzung in einer Menge von 3 bis 30 Gew.-% vorliegt und ausgewählt ist aus der Gruppe, bestehend aus Natriumstärkeglykolat, Croscarmellose, Crospovidon und Mischungen davon.

14. Verwendung von Orlistat nach Anspruch 10, wobei der Füllstoff in der Pulverzusammensetzung in einer Menge von 10 bis 80 Gew.-% vorliegt und ausgewählt ist aus der Gruppe, bestehend aus mikrokristalliner Cellulose, verkieselter mikrokristalliner Cellulose, pulverisierter Cellulose, Maisstärke, modifizierter Maisstärke, Kartoffelstärke, Lactosemonohydrat, wasserfreier Lactose, Mannit, zweibasischem Calciumphosphatdihydrat, wasserfreiem zweibasischem Calciumphosphat, Magnesiumcarbonat, Erythrit, Trehalose und Mischungen davon, vorzugsweise mikrokristalliner Cellulose mit niedrigem Feuchtigkeitsgehalt, Kartoffelstärke mit niedrigem Feuchtigkeitsgehalt oder einer Mischung davon.

15. Verwendung von Orlistat nach Anspruch 10, wobei die Pulverzusammensetzung ein oder mehrere zusätzliche pharmazeutisch annehmbare Additive umfasst, vorzugsweise ein in der Zusammensetzung vorhandenes Tensid in einer Menge von 0,1 bis 5 Gew.-%, vorzugsweise Natriumlaurylsulfat.

## Revendications

1. Procédé de fabrication de compositions pharmaceutiques sous la forme de poudre encapsulée comprenant de l'Orlistat et des additifs pharmaceutiquement acceptables choisis dans le groupe comprenant un agent de glissement et un ou deux d'une charge et d'un désintégrant, **caractérisé en ce que** le procédé comprend deux étapes : une première étape d'obtention d'une poudre pharmaceutique par mélange de l'Orlistat et d'additifs pharmaceutiquement acceptables et une seconde étape suivante d'encapsulation de la poudre ainsi obtenue.

2. Procédé selon la revendication 1, dans lequel l'Orlistat est présent dans la composition de poudre en une quantité de 30 à 70 % en poids.

3. Procédé selon la revendication 1, dans lequel l'agent de glissement est présent dans la composition de poudre en une quantité de 1 à 10 % en poids.

4. Procédé selon la revendication 1, dans lequel le désintégrant est présent dans la composition de poudre en une quantité de 3 à 30 % en poids.

5. Procédé selon la revendication 4, dans lequel le désintégrant est choisi dans le groupe constitué par le glycolate d'amidon sodique, le croscarmellose, la crospovidone et leurs mélanges.

6. Procédé selon la revendication 1, dans lequel la charge est présente dans la composition de poudre en une quantité de 10 à 80 % en poids.

7. Procédé selon la revendication 1, dans lequel la composition de poudre comprend un ou plusieurs additifs pharmaceutiquement acceptables supplémentaires.

8. Procédé selon la revendication 7, dans lequel l'additif pharmaceutiquement acceptable supplémentaire est un tensioactif présent dans la composition de poudre en une quantité de 0,1 à 5 % en poids.

9. Composition pharmaceutique sous la forme de poudre encapsulée comprenant de l'Orlistat fabriquée selon un procédé tel que défini dans l'une quelconque des revendications 1 à 8.

10. Utilisation de l'Orlistat dans un procédé de fabrication de compositions pharmaceutiques sous la forme de poudre encapsulée le procédé de fabrication comprenant une première étape de mélange de l'Orlistat et d'additifs pharmaceutiquement acceptables choisis dans le groupe comprenant un agent de glissement et un ou les deux d'une charge et d'un désintégrant, et une seconde étape suivante d'encapsulation du mélange.

11. Utilisation de l'Orlistat selon la revendication 10, dans laquelle l'Orlistat est présent dans la composition de poudre en une quantité de 30 à 70 % en poids.

12. Utilisation de l'Orlistat selon la revendication 10, dans laquelle l'agent de glissement est présent dans la composition de poudre en une quantité de 1 à 10 % en poids et est choisi dans le groupe constitué par le dioxyde de silice anhydre, le talc, le stéarate de magnésium, le stéarate de calcium, le fumarate de stéaryle sodique, le béhénate de glycéryle, le palmitostéarate de glycéryle, le laurylsulfate de sodium et leurs mélanges.

13. Utilisation de l'Orlistat selon la revendication 10, dans laquelle le désintégrant est présent dans la composition de poudre en une quantité de 3 à 30 % en poids et est choisi dans le groupe constitué par le glycolate d'amidon sodique, le croscarmellose, la crospovidone et leurs mélanges.

14. Utilisation de l'Orlistat selon la revendication 10, dans laquelle la charge est présente dans la composition de poudre en une quantité de 10 à 80 % en poids et est choisie dans le groupe constitué par la cellulose microcristalline, la cellulose microcristaline silicifiée, la cellulose en poudre, l'amidon de maïs, l'amidon de maïs modifié, l'amidon de pomme de terre, le monohydrate de lactose, le lactose anhydre, le mannitol, le phosphate de calcium dihydrate dibasique, le phosphate de calcium dibasique anhydre, le carbonate de magnésium, l'érythritol, le tréhalose et leurs mélanges, de préférence la cellulose microcristalline à faible teneur en humidité, l'amidon de maïs à faible teneur en humidité, l'amidon de pomme de terre à faible teneur en humidité ou un de leurs mélanges.

15. Utilisation de l'Orlistat selon la revendication 10, dans laquelle la composition de poudre comprend un ou plusieurs additifs pharmaceutiquement acceptables supplémentaires, de préférence un tensioactif présent dans la composition en une quantité de 0,1 à 5 % en poids, de préférence le lauryl sulfate de sodium.
